# EUROPEAN PATENT APPLICATION

(11) **EP 4 516 916 A1**
(43) Date of publication of application: **05.03.2025**
(21) Application number: 23811859.0
(22) Date of filing: 24.05.2023
(51) Int. Cl.: C12P 1/02, C12N 1/14, A23L 33/135, C12N 9/42

(54) **KOJI MOLD FERMENTATION PRODUCT WITH HIGH VITAMIN B CONTENT**

(30) Priority: 25.05.2022 JP 2022085521
(71) Applicant: Kikkoman Corporation, Noda-shi, Chiba 278-8601 (JP); Agency for Science, Technology and Research, Singapore 138632 (SG)
(72) Inventor: KAN, Eiichiro, Singapore 138667 (SG); CHEN, Yi Hsin, Singapore 138667 (SG); LIANTO, Kartikasaridian, Singapore 138667 (SG); KAWASHIMA, Tadaomi, Singapore 138667 (SG); CHOW, Yvonne, Singapore 138669 (SG); HERMANSEN, Christian, Singapore 138669 (SG); NAULCHAN, Khongsay, Singapore 138669 (SG)
(74) Representative: Kramer Barske Schmidtchen Patentanwälte PartG mbB
(86) International application number: PCT/JP2023/019411
(87) International publication number: WO 2023/228987

(57) **Abstract**

The invention relates to a *koji* mold-fermented material of a grain-derived raw material which has an arabinan concentration of 5 mg/g or less. The invention also relates to a method for producing a *koji* mold-fermented material of a grain-derived raw material including treating a grain-derived raw material with arabinase and cellulase and fermenting the treated raw material using a *koji* mold.

## Description

### TECHNICAL FIELD

The present invention relates to a *koji* mold-fermented material and a production method thereof.

### BACKGROUND ART

Vitamins are organic compounds which are necessary for keeping the human body functions normal but have to be taken from foods because the vitamins are hardly synthesized in the body. Of vitamins, vitamin B is a generic term for eight kinds of nutrients including vitamin B1, vitamin B2, vitamin B6, vitamin B12, niacin, pantothenic acid, folate and biotin.

Regarding folate, which is a kind of vitamin B, folate contained in foods is natural type polyglutamic acid form, and folate used as an additive or the like is synthetic type monoglutamic acid form. It has been reported that continuous intake of supplements containing folate of synthetic type monoglutamic acid form during pregnancy has health concerns, such as an increase in the incidence rate of asthma of the child to be born (NPL 1).

Meat is abundant in vitamins and nutrients such as protein and minerals and is a nutritionally important food material. However, because meat contains a large amount of lipids, in particular saturated fatty acids, and because the energy thereof is excessive also compared to other food materials, excessive intake of meat can cause various chronic diseases including metabolic syndrome.

Therefore, to suppress excessive intake of meat, use of a meat-like food, which appears just like a processed meat food, has been attracting attention. While a meat-like food contains less saturated fatty acids and has lower energy than meat, the meat-like food is a food using a food material containing a similar amount of protein to that of meat. Intake of a meat-like food as an alternative to a meat food can lead to prevention, improvement or the like of a lifestyle-related disease such as metabolic syndrome.

As a representative of meat-like food, a food using grains such as soybeans is processed for various applications and used for a food composition and a food.

PTL 1 discloses a method for producing *koji* or miso using a *koji* mold having high productivity of vitamin B2 or the like. Moreover, as a method for producing a novel biological material derived from bean pulp, PTL 2 discloses a method of treating soy pulp with cellulase for advancing liquefaction or gelation of the bean pulp.

### CITATION LIST

### PATENT LITERATURE

PTL 1: JP5974254B
PTL 2: JP3888539B

### NON PATENT LITERATURE

NPL 1: Melissa J. Whitrow et al., "Effect of Supplemental Folic Acid in Pregnancy on Childhood Asthma: A Prospective Birth Cohort Study", American Journal of Epidemiology, 2009, Vol. 170, No. 12, pages 1486-1493.

### SUMMARY OF INVENTION

### TECHNICAL PROBLEM

As described above, because vitamins are nutrients which have to be taken from foods and because intake of natural type vitamins is desirable, a meat-like food containing a high amount of vitamins is desired.

Although it has been reported that *koji* mold fermentation can increase B vitamins such as folate, a *koji* mold having high productivity of vitamin B which is obtained by mutation has to be used to obtain a fermented material containing vitamin B at a high content and having a high nutritional value (PTL 1). Moreover, although it is known to treat soy pulp with cellulase (PTL 2), the influence of cellulase treatment on the vitamin B amount has not been known.

Thus, an object of the invention is to obtain a *koji* mold-fermented material having a high vitamin B concentration.

### SOLUTION TO PROBLEM

As a result of extensive examination to solve the problem, the present inventors have found that the vitamin B concentration of a *koji* mold-fermented material obtained by treating a grain-derived raw material with arabinase and cellulase and then fermenting with a *koji* mold is increased compared to that of a *koji* mold-fermented material obtained without treating a grain-derived raw material with arabinase and cellulase.

The invention is as follows.
[1] A *koji* mold-fermented material of a grain-derived raw material which has an arabinan concentration of 5 mg/g or less.
[2] The *koji* mold-fermented material according to [1] above which has a vitamin B concentration of 40 µg/100 g or more.
[3] The *koji* mold-fermented material according to [2] above, wherein the vitamin B is folate.
[4] The *koji* mold-fermented material according to any one of [1] to [3] above, wherein the grain-derived raw material is a raw material derived from bean pulp.
[5] The *koji* mold-fermented material according to [4] above, wherein the raw material derived from bean pulp is soy pulp.
[6] The *koji* mold-fermented material according to any one of [1] to [3] above, wherein the *koji* mold is a *koji* mold of *Aspergillus.*
[7] A method for producing a *koji* mold-fermented material of a grain-derived raw material including treating a grain-derived raw material with arabinase and cellulase and fermenting the treated raw material using a *koji* mold.
[8] The production method according to [7] above, wherein the arabinan concentration of the *koji* mold-fermented material is 5 mg/g or less.
[9] The production method according to [7] or [8] above, wherein the ratio of arabinase and cellulase is 1:3 to 9:1 on unit basis.
[10] The production method according to [7] or [8] above, wherein the arabinan amount contained in the treated raw material has been decreased by 70 mass% or more from the arabinan amount of the grain-derived raw material before the treatment.
[11] *A koji* mold-fermented material of a grain-derived raw material obtainable by treating a grain-derived raw material with arabinase and cellulase and fermenting the treated raw material with a *koji* mold.
[12] The *koji* mold-fermented material according to [11] above which has an arabinan concentration of 5 mg/g or less.
[13] The *koji* mold-fermented material according to [11] or [12] above which has an increased vitamin B concentration compared to the vitamin B concentration of the grain-derived raw material before the treatment with arabinase and cellulase.

### ADVANTAGEOUS EFFECTS OF INVENTION

According to the production method which is an aspect of the invention, namely a method for producing a *koji* mold-fermented material of a grain-derived raw material including treating a grain-derived raw material with arabinase and cellulase and fermenting the treated raw material using a *koji* mold, a *koji* mold-fermented material having an increased vitamin B concentration, compared to that of a *koji* mold-fermented material obtained without treating a grain-derived raw material with arabinase and cellulase, can be obtained by treating a grain-derived raw material with arabinase and cellulase and then fermenting with a *koji* mold.

### BRIEF DESCRIPTION OF DRAWINGS

[FIG. 1A] FIG. 1A is a graph showing the N-acetylglucosamine amounts of *koji* mold-fermented materials which were treated with saccharolytic enzymes.
[FIG. 1B] FIG. 1B is a graph showing the folate amounts of *koji* mold-fermented materials which were treated with saccharolytic enzymes.
[FIG. 2A] FIG. 2A is a graph showing the N-acetylglucosamine amounts of *koji* mold-fermented materials which were fermented using *Aspergillus oryzae* strains.
[FIG. 2B] FIG. 2B is a graph showing the folate amounts of *koji* mold-fermented materials which were fermented using *Aspergillus oryzae* strains.
[FIG. 3A] FIG. 3A is a graph showing the N-acetylglucosamine amounts of *koji* mold-fermented materials which were fermented using *Aspergillus awamori, Aspergillus inuii, Aspergillus usamii* or *Aspergillus saitoi.*
[FIG. 3B] FIG. 3B is a graph showing the folate amounts of *koji* mold-fermented materials which were fermented using *Aspergillus awamori, Aspergillus inuii, Aspergillus usamii* or *Aspergillus saitoi.*
[FIG. 4A] FIG. 4A is a graph showing the N-acetylglucosamine amounts of *koji* mold-fermented materials obtained using soy pulp, wheat bran or corn powder as a grain-derived raw material.
[FIG. 4B] FIG. 4B is a graph showing the folate amounts of *koji* mold-fermented materials obtained using soy pulp, wheat bran or corn powder as a grain-derived raw material.
[FIG. 5A] FIG. 5A is a graph showing the relative values of folate in *koji* mold-fermented materials which were treated under conditions with different arabinase and cellulase ratios.
[FIG. 5B] FIG. 5B is a graph showing the relative values of folate in *koji* mold-fermented materials which were treated under conditions with different arabinase and cellulase ratios.
[FIG. 6] FIG. 6 is a graph showing the glucose amounts of enzymatically treated grain-derived raw material solutions.
[FIG. 7] FIG. 7 is a graph showing the viscosities of *koji* mold-fermented materials of a grain-derived raw material.

### DESCRIPTION OF EMBODIMENTS

The structures and preferable modes of the invention will be explained in further detail below.

In this specification, "A to B" indicating a range means "A or more and B or less". Moreover, in this specification, "weight" and "mass" and "weight%" and "mass%" are each regarded as synonyms.

### 1. Production Method of Koji Mold-Fermented Material of Grain-Derived Raw Material

The method for producing a *koji* mold-fermented material of a grain-derived raw material of this embodiment is a method for producing a *koji* mold-fermented material of a grain-derived raw material including treating a grain-derived raw material with arabinase and cellulase and fermenting the treated raw material using a *koji* mold.

### <Grain-Derived Raw Material>

An example of the grain-derived raw material is a material containing at least either of carbon and nitrogen. A *koji* mold can assimilate arabinose, which is a component of arabinan, efficiently, and thus the grain-derived raw material preferably contains arabinan.

Examples of the grain-derived raw material include protein-derived raw materials derived from whole soybeans, defatted soybeans, soybean protein, wheat gluten, peas, broad beans, adzuki beans and the like and starch-derived raw materials derived from wheat, barley, rye, bran, rice, rice bran, corn, starch pulp and the like. The grain-derived raw material may also be bean pulp, wheat bran, draff or corn powder. The wheat bran is the outer layer part of wheat grains. The draff is a residue remaining after the manufacture of beer and refers to a solid remaining after mixing and stirring crushed malt and hot water and removing wort by filtration. A kind thereof or a combination thereof can be used.

From the point of highly containing a carbon source and a nitrogen source which are essential for the growth of a *koji* mold, the grain-derived raw material is preferably bean pulp, further preferably soy pulp. The soy pulp is pulp of soybeans.

### <Treatment with Enzymes>

In the production method of this embodiment, the grain-derived raw material is treated with arabinase and cellulase. By treating the grain-derived raw material with arabinase and cellulase, the vitamin B content increases. Although the reason is not clear, speculated causes are high expression of a gene related to the metabolism of vitamin B due to a change in the balance of saccharides that the *koji* mold can assimilate, an increase in the amount of the primary metabolite as the substrate of vitamin B and the like.

The cellulase is specifically a hemicellulase, a glucanase, a glucosidase or the like, and examples of the hemicellulase include xylanase and the like.

Before the treatment with arabinase and cellulase, the grain-derived raw material may be subjected to a heating and pressurizing process. The heating and pressurizing process is preferably conducted using an autoclave, an extruder or a high-pressure heating tube reactor. By such a heating and pressurizing process, the enzymes can efficiently act on the grain-derived raw material.

In the production method of this embodiment, the ratio of arabinase and cellulase for treating the grain-derived raw material is preferably 1:3 to 9:1, further preferably 1:3 to 3:1, on enzyme unit (also referred to as "unit" or "U" below) basis. A ratio of arabinase and cellulase of (1 or more):3 on unit basis has an advantage of a further increase in the vitamin B amount.

In the production method of this embodiment, the grain-derived raw material is preferably treated with 4 U or more of arabinase and 1.6 U or more of cellulase per 1 g of the grain-derived raw material. The concentration of arabinase and the concentration of cellulase per 1 g of the grain-derived raw material are further preferably 4 to 15 U and 10 to 12.5 U, respectively. When the concentration of arabinase and the concentration of cellulase per 1 g of the grain-derived raw material are 4 U or more and 1.6 U or more, respectively, there is an advantage because the raw material is decomposed sufficiently and because the vitamin B amount increases, while when the concentrations are 15 U or less and 12.5 U or less, respectively, there is an advantage because the costs used in the production can be reduced.

Moreover, arabinase and cellulase in the medium substrate containing the grain-derived raw material are preferably at 250 to 900 mU/ml and 100 to 750 mU/ml, respectively.

The temperature, the pH, the treatment time and the like of the treatment of the grain-derived raw material with arabinase and cellulase can be appropriately determined considering the concentrations of arabinase and cellulase and the like. The temperature is preferably 0 to 80°C, more preferably 20 to 70°C, further preferably 30 to 60°C. Temperature in the range has an advantage because the enzymes exhibit high activities and because the raw material is decomposed efficiently. The pH is preferably 2 to 8, more preferably 3 to 7, further preferably 4 to 6. pH in the range has an advantage because the enzymes exhibit high activities and because the raw material is decomposed efficiently. The treatment time is preferably one to 72 hours, more preferably three to 24 hours, further preferably five to 20 hours. Treatment time of 72 hours or less has an advantage because the production period can be shortened. Moreover, treatment time of one hour or more has an advantage because the raw material can be decomposed sufficiently. During the treatment of the grain-derived raw material with arabinase and cellulase, the raw material and the enzymes are preferably stirred because the hydrolysis is promoted due to an increase in the contact faces of the raw material and the enzymes, for example.

When the grain-derived raw material is treated with arabinase and cellulase, the order of treatment may be any order. The grain-derived raw material which has been treated with arabinase may be treated with cellulase, or the grain-derived raw material which has been treated with cellulase may be treated with arabinase. Alternatively, both arabinase and cellulase may be added to the grain-derived raw material to treat with the enzymes simultaneously.

The arabinan amount contained in the grain-derived raw material which has been treated with arabinase and cellulase is decreased preferably by 80 mass% or more, further preferably by 90 mass% or more, from the arabinan amount of the grain-derived raw material before the treatment with arabinase and cellulase. When the arabinan amount contained in the grain-derived raw material which has been treated with arabinase and cellulase is decreased by 80 mass% or more from the arabinan amount of the grain-derived raw material before the treatment with arabinase and cellulase, there is an advantage because the arabinose amount of the medium increases and because the vitamin B amount increases due to a change in the balance of saccharides that the *koji* mold can assimilate.

Genes and transcription factors which involve in the carbohydrate metabolism of a *koji* mold are activated by various saccharides (Kagaku to Seibutsu, 2019, 57(9), p532-540). Because B vitamins are required as coenzymes in the glycolysis system (Chemico-Biological Interactions, 2006, 163(1-2), 94-112), it is believed that the production of activated vitamin B, which is required for the carbohydrate metabolism, is promoted and that the vitamin B content increases.

### <Fermentation Using Koji Mold>

In the production method of this embodiment, the grain-derived raw material which has been treated with the enzymes is fermented using a *koji* mold, and thus a *koji* mold-fermented material is produced.

Before the fermentation with the *koji* mold, to achieve conditions under which the *koji* mold grows actively, another C source or N source, metal ions or a defoaming agent for removing foams during culture may be added to the grain-derived raw material which has been treated with the enzymes. The pH may be adjusted to pH 3 to 7.

Although the kind of the *koji* mold is not particularly limited, *koji* molds of *Aspergillus* are preferable because the *koji* molds are used for food production. Of these, *Aspergillus oryzae, Aspergillus awamori, Aspergillus inuii, Aspergillus usamii, Aspergillus saitoi* and the like are preferable.

In the case *of Aspergillus oryzae,* strain ATCC1011, strain ATCC22788, strain NISL1365, strain NISL2074, strain NISL1018 and the like are preferable, and strain ATCC1011 and strain ATCC22788 are more preferable. In the case of *Aspergillus awamori,* strain JCM22312 is preferable. In the case *of Aspergillus inuii,* strain NISL1608 is preferable. In the case of *Aspergillus usamii,* strain ATCC11364 is preferable. In the case of *Aspergillus saitoi,* strain NISL1541 is preferable.

Strain ATCC1011, strain ATCC22788 and strain ATCC11364 can be acquired from The Global Bioresource Center. Strain JCM22312 can be acquired from Riken.

The spores of the *koji* mold are preferably added at 1×10⁴ spores or more, preferably at 1×10⁶ to 1×10⁸ spores, per 1 g of the grain-derived raw material which has been treated with the enzymes.

The grain-derived raw material which has been treated with the enzymes may be put into a container which can prevent contamination with harmful microorganisms and fermented with the *koji* mold in the container. Here, the container which can prevent contamination with harmful microorganisms may be a container having a structure which can disconnect the inside of the container and the external atmosphere. A sterilized polypropylene jar, a glass medium bottle or the like can be used for experimental application, and a jar fermenter having a function capable of supplying sterile air into the container, a pressure type fermentation tank or the like can be used for industrial application. Moreover, to sterilize the air, a filter which can collect 99.97% or more of dust of 0.3 µm or more, such as a HEPA filter, can be used. Moreover, during the fermentation with the *koji* mold, the mixture is preferably stirred.

The fermentation with the *koji* mold may be conducted at 10 to 40°C, preferably at 25 to 37°C, for one to five days, preferably for two to three days. Fermentation temperature in the range has an advantage because the *koji* mold grows actively. Moreover, fermentation time of five days or less has an advantage because the cost of fermentation and the environmental burden are reduced. Moreover, fermentation time of one day or more has an advantage because the *koji* mold grows sufficiently.

The amount of vitamin B contained in the *koji* mold-fermented material is preferably 150% or more on mass basis, further preferably 200% or more, of the amount of vitamin B in the grain-derived raw material before the arabinase and cellulase treatment.

The amount of vitamin B contained in the *koji* mold-fermented material which has been treated with arabinase and cellulase per dry *koji* mold-fermented material is preferably 110% or more on mass basis, further preferably 120% or more, particularly preferably 150% or more, of the amount of vitamin B contained in the *koji* mold-fermented material without treatment with enzymes such as arabinase and cellulase per dry *koji* mold-fermented material.

In the production method of this embodiment, vitamin B is preferably not added from outside. This is because consumers generally tend to dislike food additives. Moreover, practically, there are problems such as an increase in the operation steps due to vitamin B preparation and an increase in the production cost for the addition.

In the production method of this embodiment, treatment such as pulverization, pasteurization, removal of bacteria, concentration, membrane separation and drying may be conducted as post-treatment. Examples of the drying include methods such as freeze drying, drying under reduced pressure and heat drying.

The post-treatment may be conducted at either stage of after the enzymatic treatment and after the fermentation treatment, for example. Moreover, the post-treatment may be single treatment or a combination of treatments.

### 2. Koji Mold-Fermented Material of Grain-Derived Raw Material

The *koji* mold-fermented material of this embodiment is a *koji* mold-fermented material of a grain-derived raw material which has an arabinan concentration of 5 mg/g or less. The *koji* mold-fermented material refers to a material fermented with a *koji* mold.

The arabinan in the *koji* mold-fermented material of this embodiment has a polymer of arabinose as the main chain and may have another constituting sugar at a side chain or the main chain.

The arabinan concentration of the *koji* mold-fermented material of this embodiment is 5 mg/g or less. A concentration of 5 mg/g or less has an advantage of an increase in the vitamin B amount. By treating the grain-derived raw material with arabinase and cellulase, the vitamin B content increases. Although the reason is not clear, it is speculated that the metabolism of vitamin B by the *koji* mold changes because the balance of saccharides that the *koji* mold can assimilate, such as arabinose and glucose, changes due to the treatment with the enzymes. It is also believed that the carbohydrate metabolism is promoted and that the fungal growth amount increases due to an increase in vitamin B, which is a coenzyme required for the glycolysis system. The arabinan concentration can be measured, for example, using an arabinan assay kit (manufactured by Megazyme) or the like.

The *koji* mold-fermented material of this embodiment preferably has an arabinan amount of 5 mg or less per 1 g of dry fermented material.

The vitamin B in the *koji* mold-fermented material of this embodiment may be any of vitamin B1, vitamin B2, vitamin B6, vitamin B12, niacin, pantothenic acid, folate and biotin and may be a kind thereof or a combination thereof. Of these, the vitamin B in the *koji* mold-fermented material of this embodiment is particularly preferably folate.

The vitamin B concentration of the *koji* mold-fermented material of this embodiment is preferably 40 µg/100 g or more, further preferably 100 µg/100 g or more, particularly preferably 120 µg/100 g or more. Here, the vitamin B in the *koji* mold-fermented material of this embodiment above does not preferably contain added vitamin B. Added folate is the monoglutamic acid form, which is different from the polyglutamic acid form that is naturally derived folate.

The vitamin B amount of the *koji* mold-fermented material of this embodiment per 100 g of dry fermented material is preferably 40 µg or more, further preferably 100 µg or more, particularly preferably 120 µg or more.

When the *koji* mold-fermented material of this embodiment is a *koji* mold-fermented material of a grain-derived raw material obtainable by treating a grain-derived raw material with arabinase and cellulase and fermenting the treated raw material with a *koji* mold, the amount of vitamin B contained in the *koji* mold-fermented material is increased preferably by 150% or more on mass basis, further preferably by 200% or more, from the amount of vitamin B in the grain-derived raw material before the treatment with arabinase and cellulase.

The vitamin B content can be measured, for example, using a folate assay kit (manufactured by VitaFast) or the like.

N-acetylglucosamine is formed from chitin, which is a component of cell walls of *koji* molds. The production amount of N-acetylglucosamine in a certain period is in proportion to the substrate fungal concentration, and thus the *koji* mold amount of the *koji* mold-fermented material of this embodiment can be estimated by measuring N-acetylglucosamine.

In the *koji* mold-fermented material of this embodiment, the ratio of vitamin B and N-acetylglucosamine is preferably 1:1×10⁻⁵ to 1:1×10⁻³ on mass basis, more preferably 1:1×10⁻⁵ to 1:5×10⁻⁴, further preferably 1:1×10⁻⁵ to 1:3×10⁴. A ratio of 1:(1×10⁻³ or less) has an advantage because a fermented material highly containing vitamin B is obtained.

The N-acetylglucosamine content can be measured, for example, using hydrochloric acid hydrolysis, a method using a *koji* mold-solubilizing enzyme (also called *"koji* mold hydrolase"), near-infrared spectroscopy or the like. In particular, a method by hydrolyzing the fermented material with a *koji* mold hydrolase and measuring free N-acetylglucosamine by the method of JL Reissing *et al.* (Jose L. Reissing et al., "A MODIFIED COLORIMETRIC METHOD FOR THE ESTIMATION OF N-ACETYLAMINO SUGARS", Journal of Biological Chemistry, 1955, Volume 217, Issue 2, Pages 959-966) is generally used.

The *koji* mold-fermented material of this embodiment may contain salt, an *umami* component or the like.

The *koji* mold-fermented material of this embodiment can be used as a meat alternative. Moreover, the *koji* mold-fermented material of this embodiment may be subjected to a cutting process such as chopping or cutting into large pieces in advance. Moreover, by adding and kneading the *koji* mold-fermented material of this embodiment into a mixture of a plant food material and another food material such as vegetable which has been subjected to a cutting process and then subjecting the kneaded mixture to cooking, a cooked material having a meat-like flavor in the form of fried chicken, *Shigureni* (food boiled down in soy sauce and ginger), Hamburg steak, meatballs, meatloaf, *Menchi-katsu* (fried cutlet of minced meat), gyoza, shumai, wontons, spring rolls, steamed pork buns or the like is obtained. In this regard, meat may be added as another food material. The cooked material is not limited to those listed above, and examples thereof include a cooked material generally using soy sauce as a seasoning component and the like.

Moreover, the *koji* mold-fermented material of this embodiment does not preferably contain meat.

As explained above, this specification discloses the following matters.

That is, the gist of the invention is as follows.
<1> A *koji* mold-fermented material of a grain-derived raw material which has an arabinan concentration of 5 mg/g or less.
<2> The *koji* mold-fermented material according to <1> above which has a vitamin B concentration of 40 µg/100 g or more.
<3> The *koji* mold-fermented material according to <2> above, wherein the vitamin B is folate.
<4> The *koji* mold-fermented material according to any one of <1> to <3> above, wherein the grain-derived raw material is a raw material derived from bean pulp.
<5> The *koji* mold-fermented material according to <4> above, wherein the raw material derived from bean pulp is soy pulp.
<6> The *koji* mold-fermented material according to any one of <1> to <5> above, wherein the *koji* mold is a *koji* mold of *Aspergillus.*
<7> A method for producing a *koji* mold-fermented material of a grain-derived raw material including treating a grain-derived raw material with arabinase and cellulase and fermenting the treated raw material using a *koji* mold.
<8> The production method according to <7> above, wherein the arabinan concentration of the *koji* mold-fermented material is 5 mg/g or less.
<9> The production method according to <7> or <8> above, wherein the ratio of arabinase and cellulase is 1:3 to 9:1 on unit basis.
<10> The production method according to any one of <7> to < 9> above, wherein the arabinan amount contained in the treated raw material has been decreased by 70 mass% or more from the arabinan amount of the grain-derived raw material before the treatment.
<11> *A koji* mold-fermented material of a grain-derived raw material obtainable by treating a grain-derived raw material with arabinase and cellulase and fermenting the treated raw material with a *koji* mold.
<12> The *koji* mold-fermented material according to <11> above which has an arabinan concentration of 5 mg/g or less.
<13> The *koji* mold-fermented material according to <11> or <12> above which has an increased vitamin B concentration compared to the vitamin B concentration of the grain-derived raw material before the treatment with arabinase and cellulase.

### EXAMPLES

The invention is explained specifically below referring to Examples, but the invention is not limited to the Examples below unless it does not go beyond the gist thereof.

First, prior to the Examples, the materials shown below were prepared.

### (Grain-Derived Raw Materials)

As the soy pulp, a byproduct generated in the tofu or soy milk manufacture was used. As the wheat bran, a byproduct generated in the wheat flour manufacture was used. As the corn powder, POLENTA (manufactured by NUVITALITY) was used.

### (Enzymes)

As the arabinase, "PECLYVE FILTRATION" (manufactured by Soufflet biotechnologies) was used, and as the cellulase "CELLULYVE 50L" (manufactured by Soufflet biotechnologies) was used. As the pectinase, "PECTINASE ULTRA SP-L" (manufactured by Novozymes) was used. As the mannanase, "Mannanase BGM "Amano" 10" (manufactured by Amano Enzyme Inc.) was used.

### (Others)

As the reagents, commercial guaranteed reagents were used.

### [Preparation of Medium Substrates]

In Examples 2 to 8 described below, media suitable for the growth of *koji* molds were prepared by treating grain-derived raw materials with enzymes by the following procedures.

Distilled water (100 mL) was added to 6 g of a dry grain-derived raw material, and the mixture was autoclaved at 121°C for 30 minutes. An enzyme solution which was filtered with a 0.22-µm filter was added to the grain-derived raw material solution at a final concentration of 1 U/ml, and the reaction was conducted with stirring at 55°C overnight. After the reaction, pasteurization was conducted at 121°C for 30 minutes, and thus a medium substrate was obtained. The grain-derived raw material and the kinds of enzymes used are as shown in each Example.

### [Preparation of Fermented Materials]

In Examples 2 to 5, 7 and 8 described below, fermented materials were prepared by the following procedures.

Any of the *koji* molds shown in Table 1 was inoculated in maltodextrin agar medium (manufactured by Sigma), and spores were obtained. The spores were suspended in a surfactant-containing solution (Tween20 0.01%), inoculated in the medium substrate to a final concentration of 5.0×10⁵ spores/ml and cultured with shaking for two days (30°C, 180 rpm). After the culture, the fermented material was pasteurized by heating (80°C, 20 minutes). Then, the fermented material was homogenized with a homogenizer (manufactured by VIOLAMO) and freeze-dried. The kind of *koji* mold used is as shown in each Example.

### [Table 1]

**Table 1**

| Genus·Species of *Koji* Mold | Strain |
|---|---|
| *Aspergillus oryzae* | ATCC22788, ATCC1011, NISL1365, NISL2074, NISL1018 |
| *Aspergillus awamori* | JCM22312 |
| *Aspergillus inuii* | NISL1608 |
| *Aspergillus usamii* | ATCC11364 |
| *Aspergillus saitoi* | NISL1541 |

### [Measurement of N-Acetylglucosamine Amounts in Fermented Materials]

In Examples 2 to 4 described below, the N-acetylglucosamine amounts were measured by the following procedures.

The dry fermented material in an amount of 40 mg was suspended in 5 mL of 20 mM phosphate buffer (pH 7.0), and the supernatant was removed by centrifugation (3500 rpm, 10 minutes). After the washing operation was repeated three times, the material was suspended in a 2 mg/ml Yatalase (manufactured by Takara Bio Inc.) solution, and the reaction was conducted at 37°C for three hours. After the reaction, the supernatant was collected by centrifugation (3500 rpm, 10 minutes) and used for measuring the N-acetylglucosamine amount.

The N-acetylglucosamine was measured in accordance with the method of JL Reissing *et al.* As standard solutions of N-acetylglucosamine, solutions of 10, 50, 100 and 250 µg/ml were prepared. To 200 µl of the standard solutions, 20 µl of a 0.8 M boric acid solution (pH 9.1) was added, and after mixing, the reaction was conducted in a boiling bath for three minutes. After cooling the samples, 1.2 ml of a 100 mg/ml p-dimethylaminobenzaldehyde (hydrochloric acid:acetic acid = 1:7) solution was added and mixed, and then the reaction was conducted at 37°C for 20 minutes. After the reaction, the absorbances at 585 nm were measured, and a calibration curve was created. The samples of the Test Examples obtained in the Examples below were also colored in the same manner, and the N-acetylglucosamine amounts were determined based on the calibration curve.

### [Measurement of Folate Amounts of Grain-Derived Raw Materials and Fermented Materials]

In Examples 1 to 5 described below, the folate amounts were measured by the following procedures.

Using 1 g of the dry grain-derived raw material or the fermented material, the folate amount was measured by microbiological determination. Preparation was in accordance with the method of a folate assay kit (manufactured by VitaFast), and after reaction at 37°C for two days, the absorbance at 340 nm was measured.

### <Example 1>

### [Assessment of Folate Amounts of Grain-Derived Raw Materials]

The folate amounts of the soy pulp, the wheat bran and the corn powder which are shown in Test Examples 1 to 3 in Table 2 and which were subjected to neither enzymatic reaction nor *koji* mold fermentation as grain-derived raw materials were measured. It was found that the soy pulp contained folate most abundantly of the assessed grain-derived raw materials.

### [Table 2]

**Table 2**

| | Grain-Derived Raw Material | Folate (µg/raw material weight 100 g) |
|---|---|---|
| Test Example 1 | Soy Pulp | 62.8 ± 2.3 |
| Test Example 2 | Wheat Bran | 45.6 ± 3.8 |
| Test Example 3 | Corn Powder | 4.6 ± 0.3 |

### <Example 2>

### [Assessment of Growth Amounts and Folate Amounts in Saccharolytic Enzyme-Treated Soy Pulp Media]

A medium prepared using soy pulp as the grain-derived raw material was used as the substrate, and Test Examples 5 to 9 were treated with the saccharolytic enzymes shown in Table 3. The concentrations of the enzymes are the final concentrations after adding the enzyme solutions to the grain-derived raw material solutions. Then, strain NISL1018 was inoculated and cultured.

### [Table 3]

**Table 3**

| | Enzyme (U/ml) | | | | Grain-Derived Raw Material | *Koji* Mold |
|---|---|---|---|---|---|---|
| | Pectinase | Mannanase | Arabinase | Cellulase | | |
| Test Example 4 | 0 | 0 | 0 | 0 | Soy Pulp | Strain NISL1018 |
| Test Example 5 | 1 | 0 | 0 | 0 | Soy Pulp | Strain NISL1018 |
| Test Example 6 | 0 | 1 | 0 | 0 | Soy Pulp | Strain NISL1018 |
| Test Example 7 | 0 | 0 | 1 | 0 | Soy Pulp | Strain NISL1018 |
| Test Example 8 | 0 | 0 | 0 | 1 | Soy Pulp | Strain NISL1018 |
| Test Example 9 | 0 | 0 | 0.75 | 0.25 | Soy Pulp | Strain NISL1018 |

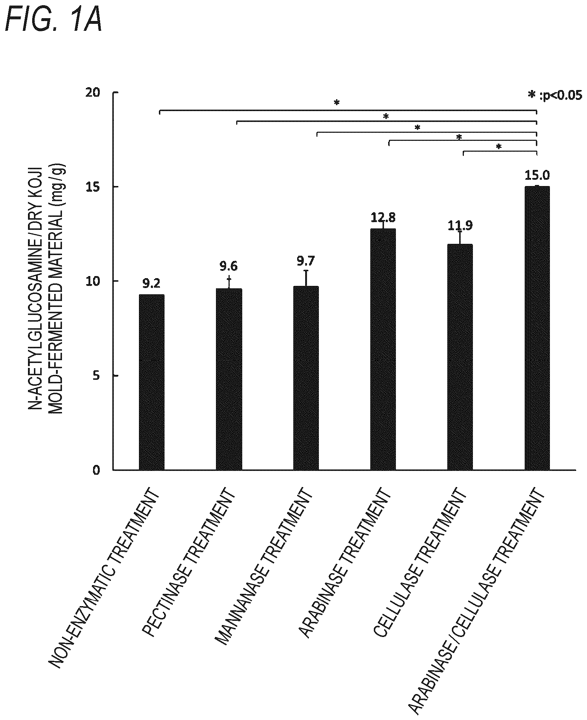

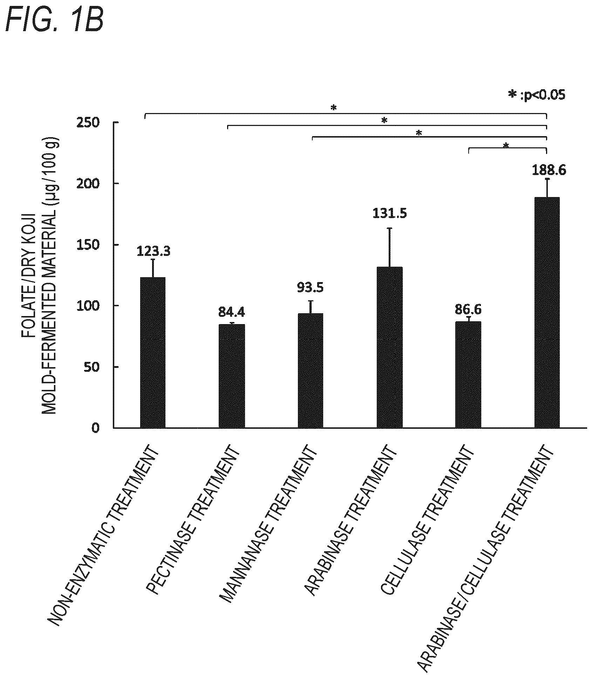

The N-acetylglucosamine amounts and the folate amounts of the obtained *koji* mold-fermented materials were assessed. The results are shown in FIG. 1A and FIG. 1B, respectively.

As a result, in Test Example 7 with arabinase treatment and Test Example 8 with cellulase treatment, an increase in the N-acetylglucosamine amount was observed. Moreover, in Test Example 9, which was treated with arabinase and cellulase, a further increase in the N-acetylglucosamine amount was observed. Furthermore, the folate amount increased significantly in Test Example 9.

It was thus suggested that the fungal amount and the folate amount of the fermented material are increased by treating the grain-derived raw material with arabinase and cellulase.

### <Example 3>

### [Assessment of Growth Amounts and Folate Amounts in Aspergillus Molds]

A medium prepared using soy pulp as the grain-derived raw material was used as the substrate, and Test Examples 11, 12, 14, 15, 17, 18, 20, 21, 23, 24, 26, 27, 29, 30, 32, 33, 35 and 36 were treated with the saccharolytic enzymes shown in Table 4 and Table 5. The concentrations of the enzymes are the final concentrations after adding the enzyme solutions to the grain-derived raw material solutions. Then, the *koji* molds shown in Table 4 and Table 5 were inoculated and cultured.

### [Table 4]

**Table 4**

| | Enzyme (U/ml) | | Grain-Derived Raw Material | *Koji* Mold |
|---|---|---|---|---|
| | Arabinase | Cellulase | | |
| Test Example 10 | 0 | 0 | Soy Pulp | Strain ATCC22788 |
| Test Example 11 | 0 | 1 | Soy Pulp | Strain ATCC22788 |
| Test Example 12 | 0.75 | 0.25 | Soy Pulp | Strain ATCC22788 |
| Test Example 13 | 0 | 0 | Soy Pulp | Strain ATCC1011 |
| Test Example 14 | 0 | 1 | Soy Pulp | Strain ATCC1011 |
| Test Example 15 | 0.75 | 0.25 | Soy Pulp | Strain ATCC1011 |
| Test Example 16 | 0 | 0 | Soy Pulp | Strain NISL1365 |
| Test Example 17 | 0 | 1 | Soy Pulp | Strain NISL1365 |
| Test Example 18 | 0.75 | 0.25 | Soy Pulp | Strain NISL1365 |
| Test Example 19 | 0 | 0 | Soy Pulp | Strain NISL2074 |
| Test Example 20 | 0 | 1 | Soy Pulp | Strain NISL2074 |
| Test Example 21 | 0.75 | 0.25 | Soy Pulp | Strain NISL2074 |
| Test Example 22 | 0 | 0 | Soy Pulp | Strain NISL1018 |
| Test Example 23 | 0 | 1 | Soy Pulp | Strain NISL1018 |
| Test Example 24 | 0.75 | 0.25 | Soy Pulp | Strain NISL1018 |

### [Table 5]

**Table 5**

| | Enzyme (U/ml) | | Grain-Derived Raw Material | *Koji* Mold |
|---|---|---|---|---|
| | Arabinase | Cellulase | | |
| Test Example 25 | 0 | 0 | Soy Pulp | Strain JCM22312 |
| Test Example 26 | 0 | 1 | Soy Pulp | Strain JCM22312 |
| Test Example 27 | 0.75 | 0.25 | Soy Pulp | Strain JCM22312 |
| Test Example 28 | 0 | 0 | Soy Pulp | Strain NISL1608 |
| Test Example 29 | 0 | 1 | Soy Pulp | Strain NISL1608 |
| Test Example 30 | 0.75 | 0.25 | Soy Pulp | Strain NISL1608 |
| Test Example 31 | 0 | 0 | Soy Pulp | Strain ATCC11364 |
| Test Example 32 | 0 | 1 | Soy Pulp | Strain ATCC11364 |
| Test Example 33 | 0.75 | 0.25 | Soy Pulp | Strain ATCC11364 |
| Test Example 34 | 0 | 0 | Soy Pulp | Strain NISL1541 |
| Test Example 35 | 0 | 1 | Soy Pulp | Strain NISL1541 |
| Test Example 36 | 0.75 | 0.25 | Soy Pulp | Strain NISL1541 |

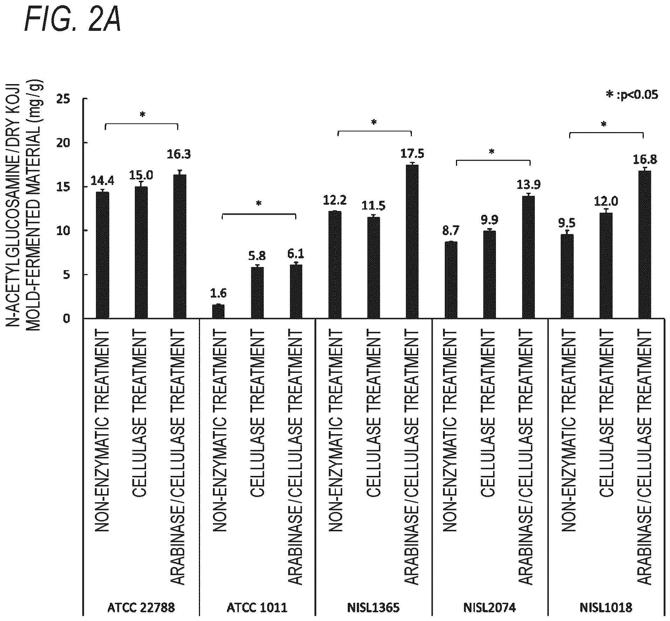

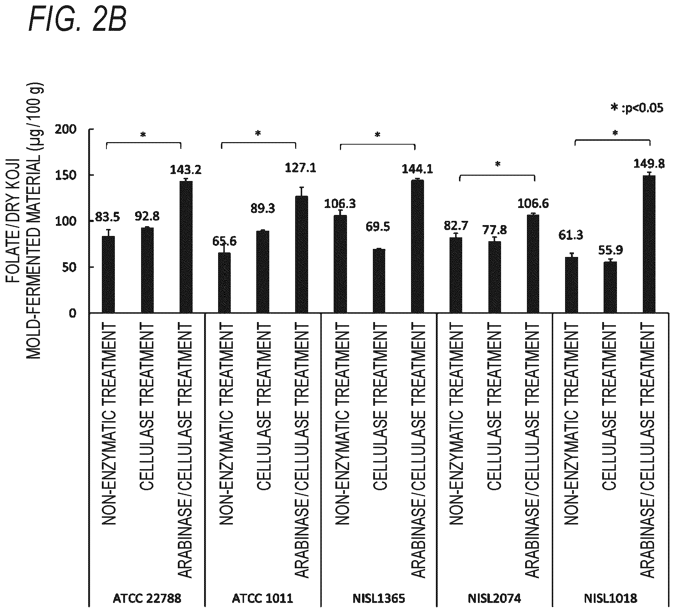

The results of assessment of the N-acetylglucosamine amounts and the folate amounts of the *koji* mold-fermented materials shown in Table 4 which were fermented using *Aspergillus oryzae* as the *koji* mold are shown in FIG. 2A and FIG. 2B, respectively. It was found that the N-acetylglucosamine amount per dry *koji* mold-fermented material increased and that the folate amount also increased with each strain by treating with arabinase and cellulase, compared to those under the conditions without the enzymatic treatment.

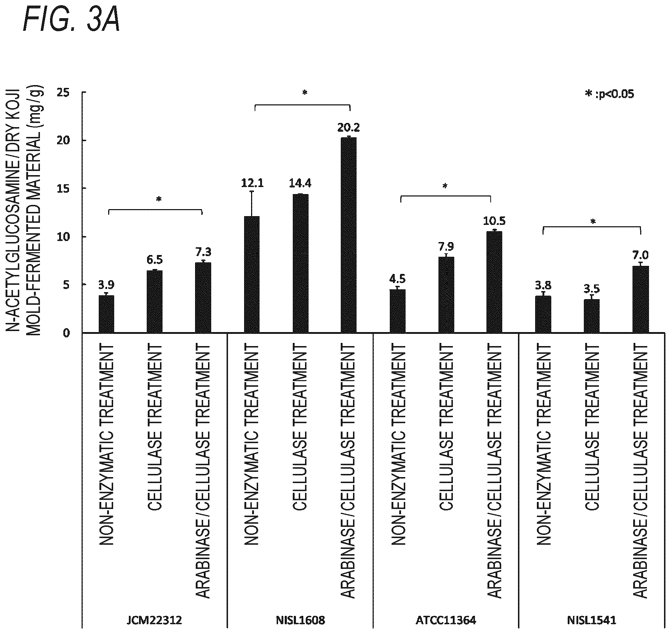

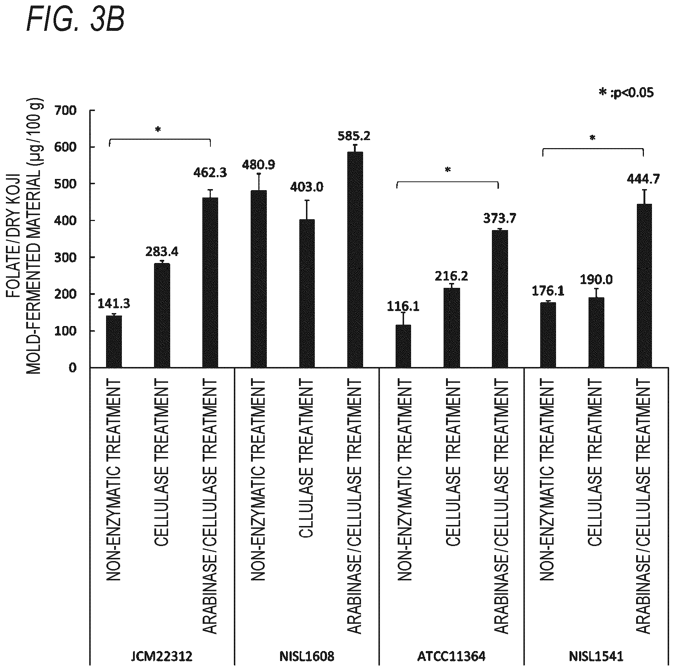

Moreover, the results of assessment of the N-acetylglucosamine amounts and the folate amounts of the *koji* mold-fermented materials shown in Table 5 which were fermented using *Aspergillus awamori, Aspergillus inuii, Aspergillus usamii* or *Aspergillus saitoi* are shown in FIG. 3A and FIG. 3B, respectively. It was found that the N-acetylglucosamine amount per dry *koji* mold-fermented material increased and that the folate amount also increased by treating with arabinase and cellulase, compared to those under the conditions without the enzymatic treatment.

### <Example 4>

### [Assessment of Growth Amounts of Koji Molds and Folate Amounts in Grain-Derived Raw Materials]

Media prepared using soy pulp, wheat bran or corn powder as the grain-derived raw materials as shown in Table 6 were used as the substrates, and Test Examples 38, 40 and 42 were treated with arabinase and cellulase. The concentrations of the enzymes are the final concentrations after adding the enzyme solutions to the grain-derived raw material solutions. Then, culture was conducted using strain ATCC1011.

### [Table 6]

**Table 6**

| | Enzyme (U/ml) | | Grain-Derived Raw Material | *Koji* Mold |
|---|---|---|---|---|
| | Arabinase | Cellulase | | |
| Test Example 37 | 0 | 0 | Soy Pulp | Strain ATCC1011 |
| Test Example 38 | 0.75 | 0.25 | Soy Pulp | Strain ATCC1011 |
| Test Example 39 | 0 | 0 | Wheat Bran | Strain ATCC1011 |
| Test Example 40 | 0.75 | 0.25 | Wheat Bran | Strain ATCC1011 |
| Test Example 41 | 0 | 0 | Corn Powder | Strain ATCC1011 |
| Test Example 42 | 0.75 | 0.25 | Corn Powder | Strain ATCC1011 |

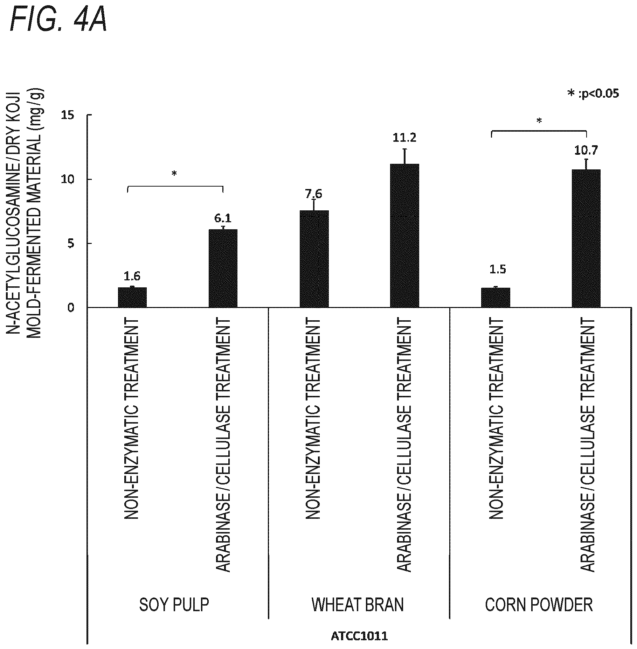

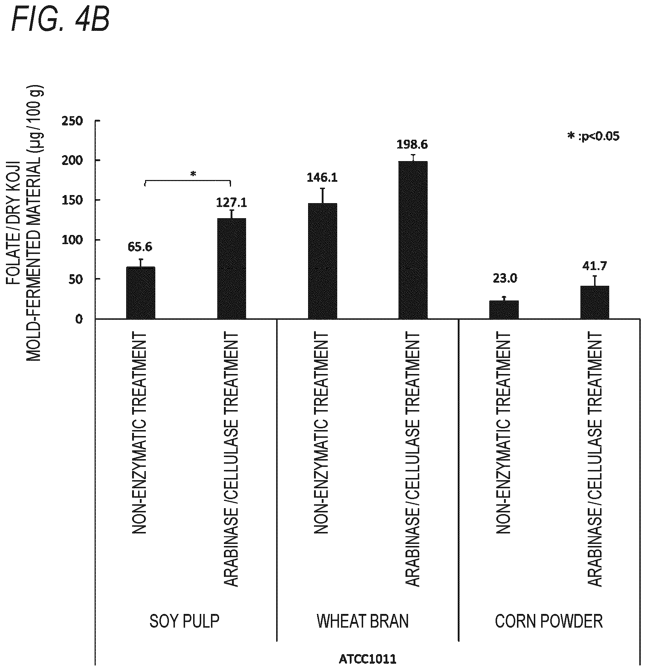

The results of assessment of the N-acetylglucosamine amounts and the folate amounts of the *koji* mold-fermented materials are shown in FIG. 4A and FIG. 4B, respectively. The N-acetylglucosamine amounts per dry *koji* mold-fermented material increased in the *koji* mold-fermented materials using all the grain-derived raw materials by treating with arabinase and cellulase, compared to those under the conditions without the enzymatic treatment. It was also found that the folate amounts increased in the *koji* mold-fermented materials using all the grain-derived raw materials by arabinase and cellulase treatment, compared to those under the conditions without the enzymatic treatment.

It was thus suggested that, when an arabinan-containing grain-derived raw material is used as the medium substrate, the folate amount of the *koji* mold-fermented material increases by treating with arabinase and cellulase, compared to the case without treatment with these enzymes.

### <Example 5>

### [Influence of Enzyme Ratio on Folate Amount]

To examine the influence on the *koji* mold amount and the folate amount of the fermented material under conditions with different ratios of arabinase and cellulase, a medium prepared using soy pulp as the grain-derived raw material was used as the substrate, and Test Examples 44 to 48 and 50 to 54 were treated with the enzymes shown in Table 7. The concentrations of the enzymes are the final concentrations after adding the enzyme solutions to the grain-derived raw material solutions. Then, culture was conducted using strain ATCC1011 or strainATCC22788.

### [Table 7]

**Table 7**

| | Enzyme (U/ml) | | Grain-Derived Raw Material | *Koji* Mold |
|---|---|---|---|---|
| | Arabinase | Cellulase | | |
| Test Example 43 | 0 | 0 | Soy Pulp | Strain ATCC1011 |
| Test Example 44 | 0 | 1 | Soy Pulp | Strain ATCC1011 |
| Test Example 45 | 0.25 | 0.75 | Soy Pulp | Strain ATCC1011 |
| Test Example 46 | 0.75 | 0.25 | Soy Pulp | Strain ATCC1011 |
| Test Example 47 | 0.9 | 0.1 | Soy Pulp | Strain ATCC1011 |
| Test Example 48 | 1 | 0 | Soy Pulp | Strain ATCC1011 |
| Test Example 49 | 0 | 0 | Soy Pulp | Strain ATCC22788 |
| Test Example 50 | 0 | 1 | Soy Pulp | Strain ATCC22788 |
| Test Example 51 | 0.25 | 0.75 | Soy Pulp | Strain ATCC22788 |
| Test Example 52 | 0.75 | 0.25 | Soy Pulp | Strain ATCC22788 |
| Test Example 53 | 0.9 | 0.1 | Soy Pulp | Strain ATCC22788 |
| Test Example 54 | 1 | 0 | Soy Pulp | Strain ATCC22788 |

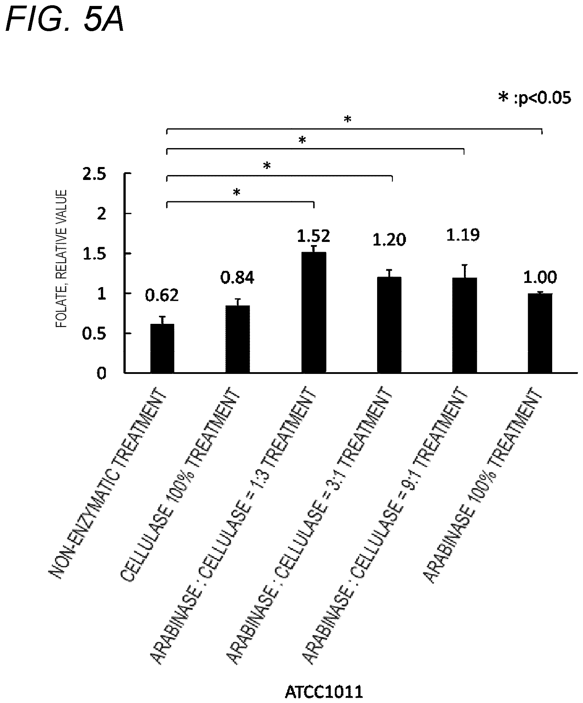

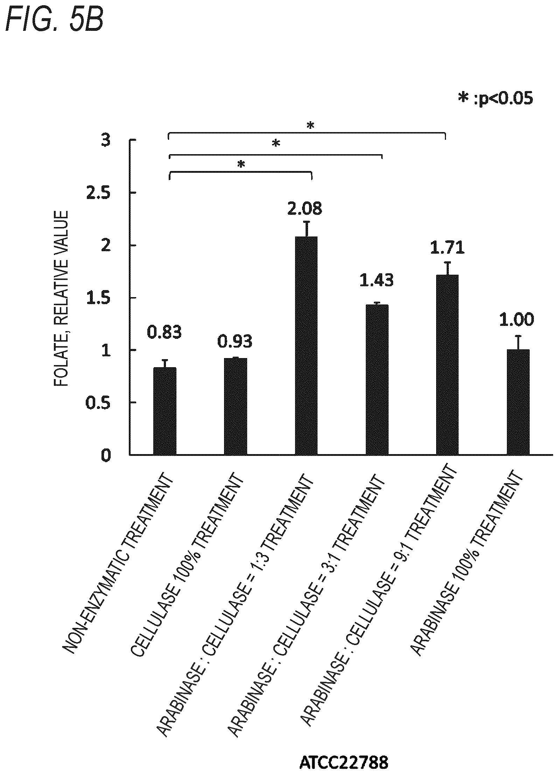

The results of assessment of the folate amounts of the *koji* mold-fermented materials which were fermented with strain ATCC1011 are shown in FIG. 5A, and the results of assessment of the folate amounts of the *koji* mold-fermented materials which were fermented with strain ATCC22788 are shown in FIG. 5B. The values of folate in the *koji* mold-fermented materials which were treated with arabinase alone were regarded as 1, and the values of the other Test Examples were shown with the relative values. In all the Test Examples in which the ratio of arabinase and cellulase was 1:3, 3:1 or 9:1 on unit basis, the folate amounts increased compared to those of the Test Examples which were treated with arabinase alone or cellulase alone. It was thus found that the folate amount of the *koji* mold-fermented material is increased by treating with enzymes having a ratio of arabinase and cellulase of 1:3 to 9:1 on unit basis.

### <Example 6>

### [Glucose Amount Measurement]

Because cellulose in the substrate is decomposed into glucose by cellulase treatment, the glucose concentrations before the culture were measured to observe the influence of the glucose amount before the culture on the folate amount.

A medium prepared using soy pulp as the grain-derived raw material was used as the substrate, and Test Examples 56 to 60 were treated with the enzymes shown in Table 8. The concentrations of the enzymes are the final concentrations after adding the enzyme solutions to the grain-derived raw material solutions. The enzymatically treated solutions in a volume of 0.2 ml were taken and centrifuged at 14,000 rpm for 10 minutes, and the supernatants were collected. The glucose concentrations of the obtained supernatants were measured using YSI Biochemistry Analyzer (manufactured by YSI Life Science).

### [Table 8]

**Table 8**

| | Enzyme (U/ml) | | Grain-Derived Raw Material |
|---|---|---|---|
| | Arabinase | Cellulase | |
| Test Example 55 | 0 | 0 | Soy Pulp |
| Test Example 56 | 0 | 1 | Soy Pulp |
| Test Example 57 | 0.25 | 0.75 | Soy Pulp |
| Test Example 58 | 0.75 | 0.25 | Soy Pulp |
| Test Example 59 | 0.9 | 0.1 | Soy Pulp |
| Test Example 60 | 1 | 0 | Soy Pulp |

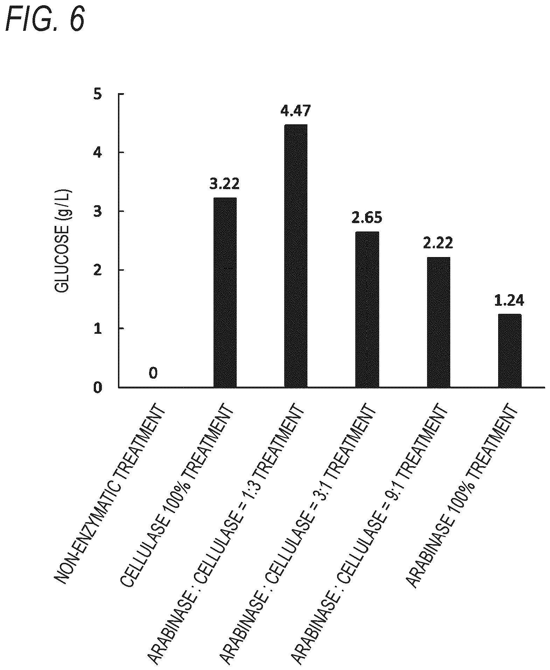

The glucose concentrations of the Test Examples are shown in FIG. 6. It was found that the glucose concentration increased by enzymatically treating with arabinase and cellulase compared to the case with arabinase treatment alone. It was thus suggested that the glucose concentration of the medium rises with arabinase and cellulase treatment. Glucose is a saccharide which a *koji* mold can assimilate, and it is speculated that the metabolism of vitamin B by the *koji* mold changes because the balance of saccharides that the *koji* mold can assimilate changes and that vitamin B in the *koji* mold-fermented material increases.

### <Example 7>

### [Arabinan Amount Measurement]

A medium prepared using soy pulp as the grain-derived raw material was used as the substrate, and Test Examples 62 to 67 were treated with the enzymes shown in Table 9. The concentrations of the enzymes are the final concentrations after adding the enzyme solutions to the grain-derived raw material solutions. Then, Test Examples 65 to 67 were cultured using strain ATCC22788.

The arabinan amounts of Test Examples 61 to 67 were measured. An arabinan assay kit (manufactured by Megazyme) was used for measuring the arabinan amounts.

In Test Examples 61 to 64, the medium supernatants were collected as unfermented samples, and the arabinan-containing filtrates from which free arabinose was removed by passing through the attached PD-10 column were used as the samples. In Test Examples 65 to 67, the samples obtained by freeze-drying the fermented materials were suspended in distilled water and used after similarly passing through the PD-10 column.

The kit contains endoarabinase, α-L-arabinofuranosidase and galactose mutarotase. Arabinan is converted into β-L-arabinose by the enzymes. In the presence of NAD⁺, L-arabinonic acid and NADH are generated from β-L-arabinose by β-galactose dehydrogenase. By measuring the absorbances at 340 nm, the free NADH amounts were measured, and the arabinan concentrations were determined. The measured arabinan concentrations are shown in Table 9. As the arabinan concentrations in Table 9, the arabinan weights per dried raw material weight are shown for Test Examples 61 to 64, and the arabinan weights per dried *koji* mold-fermented material weight are shown in Test Examples 65 to 67.

### [Table 9]

**Table 9**

| | Enzyme (U/ml) | | Grain-Derived Raw Material | *Koji* Mold | Arabinan (mg/dry weight g) |
|---|---|---|---|---|---|
| | Arabinase | Cellulase | | | |
| Test Example 61 | 0 | 0 | Soy Pulp | None | 38.0 |
| Test Example 62 | 0.25 | 0.75 | Soy Pulp | None | 8.4 |
| Test Example 63 | 0.9 | 0.1 | Soy Pulp | None | 4.6 |
| Test Example 64 | 1 | 0 | Soy Pulp | None | 3.4 |
| Test Example 65 | 0.25 | 0.75 | Soy Pulp | Strain ATCC22788 | 5.1 |
| Test Example 66 | 0.9 | 0.1 | Soy Pulp | Strain ATCC22788 | 3.1 |
| Test Example 67 | 1 | 0 | Soy Pulp | Strain ATCC22788 | 3.7 |

In Test Example 61, in which neither of the enzymatic reactions with arabinase and cellulase was conducted and in which fermentation with the *koji* mold was not conducted, the arabinan concentration was 38.0 mg/g per raw material weight. On the other hand, in Test Example 67, which was treated with 1 U/ml of arabinase and fermented with the *koji* mold, the arabinan amount was 3.7 mg/g per dry *koji* mold-fermented material weight. Moreover, in Test Example 65, which was treated with arabinase and cellulase at a ratio of 1:3 on unit basis and fermented with the *koji* mold, the arabinan amount was 5.1 mg/g per dry *koji* mold-fermented material weight, and in Test Example 66, which was treated with arabinase and cellulase at a ratio of 9:1 on unit basis and fermented with the *koji* mold, the arabinan amount was 3.1 mg/g per dry *koji* mold fermented material weight. It was thus found that the arabinan amounts of the *koji* mold-fermented materials using soy pulp treated with enzymes per dry *koji* mold-fermented material weight were 3.1 mg/g to 5.1 mg/g.

### <Example 8>

### [Measurement of Culture Viscosities]

A medium prepared using soy pulp as the grain-derived raw material was used as the substrate and treated with the enzymes shown in Table 10. The concentrations of the enzymes are the final concentrations after adding the enzyme solutions to the grain-derived raw material solutions. Then, culture was conducted using strain ATCC22788.

### [Table 10]

**Table 10**

| | Enzyme (U/ml) | | Grain-derived raw material | *Koji* Mold |
|---|---|---|---|---|
| | Arabinase | Cellulase | | |
| Test Example 68 | 0 | 0 | Soy Pulp | Strain ATCC22788 |
| Test Example 69 | 1 | 0 | Soy Pulp | Strain ATCC22788 |
| Test Example 70 | 0 | 1 | Soy Pulp | Strain ATCC22788 |
| Test Example 71 | 0.75 | 0.25 | Soy Pulp | Strain ATCC22788 |

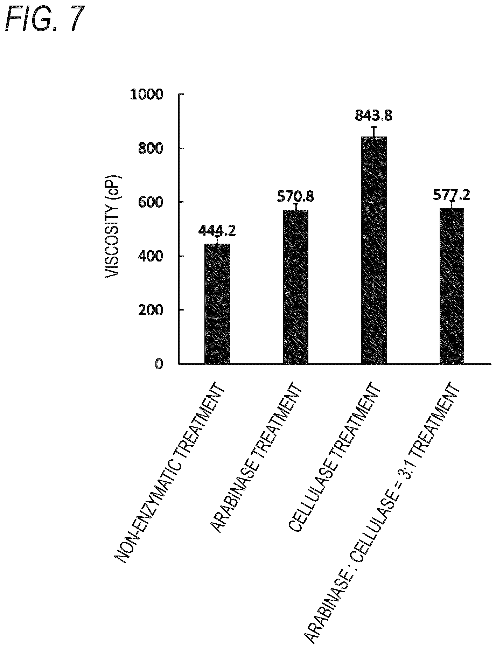

The viscosities of the samples after the culture were measured. For measuring the viscosities, 50 ml of the cultures after culturing in a 100-ml flask were collected, and a viscometer (TVB-10M, manufactured by Toki Sangyo Co., Ltd) was used. The rotor used was M3, and the stirring rate used was 60 rpm. The results are shown in Fig. 7.

It was found that, while the viscosity of Test Example 70, which was treated with cellulase, increased compared to that of Test Example 68 without the enzymatic treatment, the viscosity of Test Example 71, which was treated with arabinase and cellulase, did not increase largely. That the viscosity does not increase has an advantage in view of a decrease in the yield rate and handling during collection of the final product of the fermented material.

### INDUSTRIAL APPLICABILITY

The *koji* mold-fermented material of this embodiment can be used as a meat alternative or another food material.

Although embodiments have been explained above, it is needless to mention that the invention is not limited to the examples. It is obvious that one skilled in the art can reach modified examples or corrected examples within the scope described in the claims, and it is understood that the examples of course belong to the technical scope of the invention. The constituent features in the embodiments may be combined freely within the scope which does not deviate from the contents of the invention.

The present application is based on a Japanese patent application filed on May 25, 2022 (patent application No. 2022-085521), and the contents thereof are incorporated in the present application by reference.

## Claims

1. A *koji* mold-fermented material of a grain-derived raw material which has an arabinan concentration of 5 mg/g or less.

2. The *koji* mold-fermented material according to claim 1 which has a vitamin B concentration of 40 µg/100 g or more.

3. The *koji* mold-fermented material according to claim 2, wherein the vitamin B is folate.

4. The *koji* mold-fermented material according to any one of claims 1 to 3, wherein the grain-derived raw material is a raw material derived from bean pulp.

5. The *koji* mold-fermented material according to claim 4, wherein the raw material derived from bean pulp is soy pulp.

6. The *koji* mold-fermented material according to any one of claims 1 to 3, wherein the *koji* mold is a *koji* mold *of Aspergillus.*

7. A method for producing a *koji* mold-fermented material of a grain- derived raw material including treating a grain- derived raw material with arabinase and cellulase and fermenting the treated raw material using a *koji* mold.

8. The production method according to claim 7, wherein the arabinan concentration of the *koji* mold-fermented material is 5 mg/g or less.

9. The production method according to claim 7 or 8, wherein the ratio of arabinase and cellulase is 1:3 to 9:1 on unit basis.

10. The production method according to claim 7 or 8, wherein the arabinan amount contained in the treated raw material has been decreased by 70 mass% or more from the arabinan amount of the grain- derived raw material before the treatment.

11. A *koji* mold-fermented material of a grain- derived raw material obtainable by treating a grain- derived raw material with arabinase and cellulase and fermenting the treated raw material with a *koji* mold.

12. The *koji* mold-fermented material according to claim 11 which has an arabinan concentration of 5 mg/g or less.

13. The *koji* mold-fermented material according to claim 11 or 12 which has an increased vitamin B concentration compared to the vitamin B concentration of the grain-derived raw material before the treatment with arabinase and cellulase.
